(19) Europäisches Patentamt
European Patent Office
Office européen des brevets

(11) Publication number: **0 080 847**
**A2**

(12) # EUROPEAN PATENT APPLICATION

(21) Application number: 82306206.2

(22) Date of filing: 22.11.82

(51) Int. Cl.$^3$: C 07 D 405/06
//A61K31/44

(30) Priority: 27.11.81 US 325518
27.11.81 US 325517

(43) Date of publication of application:
08.06.83 Bulletin 83/23

(84) Designated Contracting States:
BE CH DE FR GB IT LI LU NL SE

(71) Applicant: ELI LILLY AND COMPANY
307, East McCarty Street
Indianapolis Indiana 46285(US)

(72) Inventor: Booher, Richard Nolan
6886, Hillcrest Court
Indianapolis Indiana 46227(US)

(72) Inventor: Farkas, Eugene
7832 N, Graham Road
Indianapolis Indiana 46250(US)

(74) Representative: Crowther, Terence Roger et al,
Erl Wood Manor
Windlesham Surrey GU20 6PH(GB)

(54) **Improvements in or relating to the synthesis of dioxane analgesics.**

(57) A series of 3-pyridyl-dialkylamino-m-dioxanylmethanes, which are analgesics, can be prepared by forming an enamine of a pyridylcarbonyldioxane, and reducing the enamine.

EP 0 080 847 A2

# IMPROVEMENTS IN OR RELATING TO THE SYNTHESIS OF DIOXANE ANALGESICS

This invention relates to an improved synthesis of N,N-dialkyl-α-(3-pyridyl)-*m*-dioxane-5-methylamines, the analgesic properties of which have been described in U.S. Patent No. 3,905,987.

According to the present invention there is provided a process for preparing a compound of formula (I):

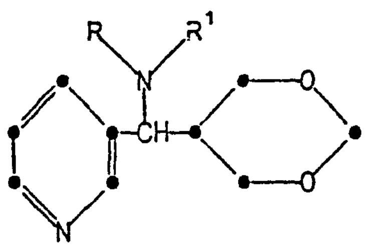

(I)

wherein R and $R^1$ are independently $C_1$-$C_4$ alkyl; or a pharmaceutically-acceptable acid-addition salt thereof.

which process comprises reacting a pyridylcarbonyldioxane of formula:

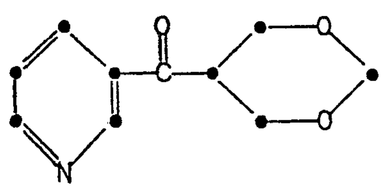

with a secondary amine of formula:

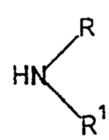

in the presence of titanium tetrachloride to prepare an enamine of the formula

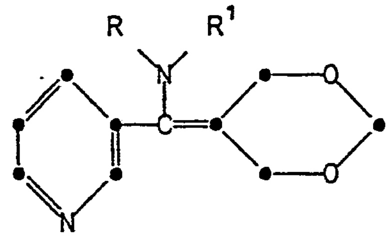

and reducing the enamine.

In this document, all temperatures are expressed in degrees Celsius. The general chemical terms have their usual meanings; for example, $C_1$-$C_4$ alkyl includes such groups as methyl, ethyl, propyl, isopropyl, *t*-butyl, or *n*-butyl.

The nature of the products produced by the process of this invention is believed to be clear from the foregoing description. However, the following typical products which may be made by this process are mentioned to insure comprehension.

N-butyl-N-ethyl-α-(3-pyridyl)-*m*-dioxane-5-methylamine

N,N-dipropyl-α-(3-pyridyl)-*m*-dioxane-5-methylamine

N-ethyl-N-isopropyl-α-(3-pyridyl)-*m*-dioxane-5-methylamine

N-*s*-butyl-N-isopropyl-α-(3-pyridyl)-*m*-dioxane-5-methylamine

N-*t*-butyl-N-ethyl-α-(3-pyridyl)-*m*-dioxane-5-methylamine

N-butyl-N-*s*-butyl-α-(3-pyridyl)-*m*-dioxane-5-methylamine

N-isobutyl-N-methyl-α-(3-pyridyl)-m-dioxane-5-methylamine

The products wherein R and $R^1$ are methyl are preferred.

Booher in U.S. Patent No. 3,905,987 taught that the compounds prepared by the present process may be used in the form of acid-addition salts. Accordingly, it will be understood that the products of the present invention may be isolated and recovered in the form of such salts, such as, especially, hydrohalides and such pharmaceutically acceptable salts. The compounds can form diacid salts of the stronger acids, such as the hydrohalides. The most pharmaceutically important product which can be prepared utilising the process of this invention is l-N,N-dimethyl-α-(3-pyridyl)-m-dioxane-5-methylamine, hydrochloride, known as doxpicomine.

The novel pyridylcarbonyldioxane starting material can be prepared by condensing 3-acetylpyridine with a polymer of formaldehyde in the presence of boron trifluoride. The condensation goes best in an acid reaction mixture, preferably using acetic acid or another alkanoic acid as the solvent. It is most preferred to use from 2-4 moles of boron trifluoride etherate, and from 2-5 moles of paraformaldehyde at temperatures in the range of from 50-80°.

An alternative synthesis of the pyridylcarbonyldioxane involves the reaction of 5-cyano-m-dioxane with 3-lithiopyridine. The reaction is analogous to a Grignard reaction, and is preferably run in an ether, especially diethyl ether or tetrahydrofuran,

at low temperatures in the range of from -100° to 0°, preferably from -80° to -20°.

The 5-cyano-m-dioxane used above can be prepared by the condensation of a polymer of formaldehyde with a substituted propanediol of the formula

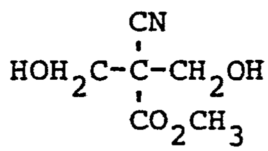

to obtain a dioxane of the formula

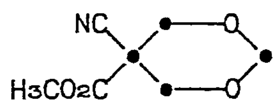

The condensation may be effected in the presence of boron trifluoride or a strong organic acid such as methane- or toluenesulfonic acid.

The dioxane above can then be decarboxylated, as by water-dimethyl sulfoxide, according to the method of Krapcho et al., J. Org. Chem. 43, 138-46 (1978).

The first step of the process of this invention involves the reaction of the pyridylcarbonyldioxane with a secondary amine having the desired R and $R^1$ groups to form the enamine intermediate. The process is carried out in the presence of titanium tetrachloride. The examples below show that the preferred amount of titanium tetrachloride lies in the range of from 0.5 mole per mole of starting compound to 0.8 mole per mole of starting compound. It is most preferred to use from 0.5 to 0.6 mole of titanium tetrachloride per mole of starting compound, but it will be

understood that the optimum amount of the catalyst will vary, inter alia, depending on the compound being prepared. For example, in some instances it may be advantageous to optimize the speed of the reaction, to achieve greater throughput, and in other instances it may be advantageous to optimize the yield of the process, regardless of its speed. Obviously, the exact optimum conditions will vary depending on the objective of the optimization. Larger amounts of titanium tetrachloride can be used if desired, even up to several moles per mole of starting compound.

It is usually advisable to use a substantial excess of the secondary amine reactant. It has been found that better yields are obtained when a large excess of amine is added, and further, of course, the amine is ordinarily much less expensive than is the pyridylcarbonyldioxane. Accordingly, the preferred amount of the amine is in the range of 3-6 moles per mole of the dioxane, more preferably 5-6 moles. However, amounts of the amine up to an excess as large as 10 moles per mole of dioxane, or even greater, may be used as may be most effective, depending on the goal of a given process.

It is preferred to conduct the enamine reaction in aromatic hydrocarbon solvents such as benzene or toluene, which solvents have been found to facilitate the isolation of the enamine for the next step. Xylene is somewhat less preferred as a solvent. The process can be carried out in other solvents as well as in the preferred solvents, including such commonly used inert reaction solvents as halogenated solvents including

dichloromethane, chloroform, chlorobenzene, and the various dichlorobenzenes and chlorotoluenes.

The enamine reaction gives economically complete yields in relatively short periods of time, depending, of course, on the temperature. It has been found advantageous to use a period of 30 minutes at about 0° followed by 30 minutes at ambient temperature. It will be understood that temperatures from about -25° to about ambient temperature may be used as may be advantageous in a given situation. It is more preferred to use temperatures from about -10° to about 20° while the reactants and the catalyst are being combined; the reaction mixture may be warmed to about ambient temperature after it is complete in the preferred practice of this invention. Higher temperatures, above ambient temperature, may be used if desired but are likely to result in depressed yields.

The enamine can be reduced by any convenient means to form the desired product. It is not necessary to purify the enamine before reducing it; adequately pure enamine is obtained by merely filtering the titanium dioxide and amine hydrochloride out of the reaction mixture, and evaporating the filtrate to dryness.

Conventional reduction methods, such as are commonly used for the reduction of enamine compounds in general, work quite well in this process. For example, a preferred method of reduction is hydrogenation in the presence of a noble metal catalyst, especially a platinum catalyst on a carbon carrier. Palladium catalysts are also quite effective; other commonly used hydrogenation catalysts, such as iridium, ruthenium and the

like may be employed in the conventional manner. Relatively low hydrogen pressures, in the range of about 1-5 atmospheres, are quite adequate. As usual, the common inert reaction solvents may be used for hydrogenations, especially alkanols.

Another preferred exemplary method of reduction is by means of a hydride, especially a borohydride or cyanoborohydride. More particularly, sodium borohydride and sodium cyanoborohydride are preferred reducing agents. Other chemical reducing agents can also be used, however, such as lithium aluminum hydride, sodium bis(2-methoxyethoxy)aluminum hydride and the like..

It is preferred to use a substantial excess of the chemical reducing agent, in the range of from 2 to 6 moles per mole of enamine. However, an amount approximating the stoichiometric amount, or a large excess, even up to 10 moles or more, may also be used.

Chemical reductions are usually carried out in an acid environment, and it is preferred to use a small amount of acetic acid in the mixture. Whether acetic is used or some other acid, it is necessary only to add a sufficient amount of acid to ensure that the mixture is acid during the reduction. Amounts in the range of from about the stoichiometric amount to 2 or 3 moles per mole of enamine are adequate.

In general, reductions are carried out in an inert organic solvent. Any appropriate solvent may be used, of which alkanols, especially methanol, ethanol and propanol, are preferred. Other inert organic solvents such as were discussed above may be used, however, as may be appropriate in a given situation.

Typically chemical reductions are carried out at about ambient temperature; the temperature at which this step is performed, however, may be varied over a wide range, as may be convenient in the circumstances.

The compounds which are the products of this invention are optically active, and the l-isomers are usually preferred. The mixtures of optical isomers which are prepared by the process of this invention are readily resolved by forming mixtures of salts with optically active acids and selectively crystallizing.

The following Examples are offered to facilitate the understanding of the invention.

Example 1

5-(3-Pyridylcarbonyl)-m-dioxane

A 60.6 g. portion of 3-acetylpyridine was combined with 54 g. of paraformaldehyde and 250 ml. of acetic acid. The mixture was cooled in an ice bath, and 184 ml. (1.5 moles) of boron trifluoride etherate was added dropwise with stirring, over a period of about 40 minutes. The temperature of the mixture ranged from 15° to 26° during the addition. The mixture was then stirred under reflux, at about 67°, for 2 hours 15 minutes, and was then cooled slightly. The volatile portions and most of the acetic acid were removed under vacuum, and the remaining mixture was rinsed with water to a large beaker and was made basic with ammonium hydroxide. The basic mixture was filtered through glass wool, and the filtrate was extracted with chloroform. The organic layer was then washed with saturated aqueous sodium chloride, and was

dried over magnesium sulfate and filtered. The filtrate was evaporated under vacuum to obtain 128 g. of crude product, which was recrystallized from isopropanol. The total yield was 59.1 g. of purified product, the first crop of which had a melting point of 98-100°.

Example 2

5-(3-Pyridyl)(dimethylamino)methylene-*m*-dioxane

Ten g. of the ketone of Example 1 was added to a flask and dissolved in 150 ml. of benzene. The solution was cooled to 5-10°, and 20.6 ml. of dry dimethylamine was added. A cold solution of 3.4 ml. of titanium tetrachloride in 50 ml. of benzene was added dropwise with stirring while the reaction mixture was maintained at a temperature below 20°. When the addition was complete, the mixture was stirred in an ice bath for 30 minutes, and then at ambient temperature for 30 minutes more. The mixture was then filtered and the filter cake was washed with benzene. The filtrate was then evaporated under vacuum to obtain a viscous residue weighing 10.8 g. Nuclear magnetic resonance analysis of the residue in $CDCl_3$ showed characteristic peaks at $\delta$2.77 (s, 6H); 4.12 (s, 2H); 4.68 (s, 2H); and 4.99 (s, 2H); indicating the presence of the desired enamine.

Example 3

5-(3-Pyridyl)(dimethylamino)methylene-*m*-dioxane

An 11.6 g. portion of the ketone of Example 1 was dissolved in 180 ml. of toluene, cooled to about

5°, and combined with 24 ml. of dimethylamine. To the mixture was added dropwise with stirring, 4.0 ml. of titanium tetrachloride in 60 ml. of cold toluene, while the reaction mixture was kept below 15°. The addition took about 15 minutes. After the addition, the mixture was stirred for 30 minutes in the ice bath and then for 30 minutes at ambient temperature. The solids were filtered away and washed, and the filtrate was evaporated to obtain 11.8 g. of crude oily product, identical to the product of Example 2 by nmr analysis.

## Example 4

5-(3-Pyridyl)(dimethylamino)methylene-*m*-dioxane

Five g. of the ketone of Example 1 was dissolved in 75 ml. of dichloromethane, and 10.3 ml. of dimethylamine was added. To the solution in an ice bath was added 1.7 ml. of titanium tetrachloride, dissolved in 25 ml. of cold dichloromethane, dropwise with stirring. When the addition was complete, the mixture was stirred for 30 minutes in the ice bath and then for 30 minutes at ambient temperature, and was then filtered. It was necessary to filter several times, because dimethylamine hydrochloride precipitated slowly from the solution. When it stopped precipitating, the filtrate was evaporated under vacuum to obtain 6.4 g. of impure product, nmr analysis of which indicated the presence of the desired enamine, identical to the product of Example 2.

Example 5

5-(3-Pyridyl)(dimethylamino)methylene-*m*-dioxane

One g. of the ketone of Example 1 was dissolved in 15 ml. of chlorobenzene, and mixed with 2.1 ml. of dimethylamine. To the solution, in an ice bath, was added 0.34 ml. of titanium tetrachloride dissolved in 10 ml. of cold chlorobenzene, dropwise with stirring while the temperature was held below 20°. The mixture was then stirred for 30 minutes in the ice bath and 30 minutes at ambient temperature, and was filtered. The filtrate was evaporated under vacuum to obtain a residue of impure product, which was found by nmr analysis to consist of the desired enamine of Example 2 with only small amounts of impurities.

Example 6

5-(3-Pyridyl)(dimethylamino)methylene-*m*-dioxane

Two g. of the ketone of Example 1 was dissolved in 30 ml. of benzene, and combined with 2.7 ml. of dimethylamine. To the cold solution, held below 10°, was added dropwise with stirring a cold solution of 0.68 ml. of titanium tetrachloride in 10 ml. of cold benzene. The temperature of the mixture was kept below 20° while the addition was made. The mixture was then stirred for 30 minutes in the ice bath and for 30 minutes at ambient temperature, and was filtered and the filtrate was evaporated to obtain 1.9 g. of impure product, containing about 50% of the desired enamine by nmr analysis.

Example 7

N,N-Dimethyl-α-(3-pyridyl)-*m*-dioxane-5-methylamine

A 5.19 g. portion of the enamine prepared above was dissolved in 65 ml. of denatured ethanol, and combined with 0.52 g. of 5% platinum on carbon hydrogenation catalyst in a low pressure hydrogenation vessel. The system was flushed with hydrogen and then charged with hydrogen at 3.8 kg./cm.$^2$. The vessel was shaken at ambient temperature for 5.5 hours, after which the pressure had dropped by 0.35 kg./cm.$^2$. The mixture was then removed from the vessel and filtered, and the filtrate was evaporated under vacuum to obtain 5.08 g. of viscous oil, which was identified by nmr analysis in $CDCl_3$ as follows: δ8.21 (m, 2H), 7.35 (m, 2H); 4.91 (d, 1H); 4.63 (d, 1H); 2.10 (s, 6H).

Example 8

N,N-Dimethyl-α-(3-pyridyl)-*m*-dioxane-5-methylamine

A 5.1 g. portion of the enamine prepared above was dissolved in 100 ml. of denatured ethanol and cooled, and 3 ml. of dimethylamine was added to the solution. The hydrogenation bottle containing the mixture was flushed with nitrogen, and 0.4 g. of 10% palladium on carbon hydrogenation catalyst was added to it. The vessel was then charged with hydrogen at 3.15 kg./cm.$^2$ and shaken for 16 hours. The catalyst was then filtered away and the filtrate was evaporated to dryness to obtain 4.8 g. of impure product. Nmr

analysis showed that the product consisted substantially of the desired compound, and contained very little or no enamine.

Example 9

N,N-Dimethyl-α-(3-pyridyl)-*m*-dioxane-5-methylamine

To a flask were added 2.0 g. of the enamine prepared above, 30 ml. of isopropanol and 0.68 g. of sodium borohydride in 30 ml. of ethanol. To the mixture was added with stirring in an ice bath, 1 ml. of acetic acid dissolved in 5 ml. of denatured ethanol. The addition was made dropwise, but rapidly. The mixture was then stirred for one hour in the ice bath, and was then evaporated under vacuum. The residue was dissolved in water, made basic with ammonium hydroxide and extracted three times with 60 ml. portions of dichloromethane. The combined organic layers were washed with brine, dried over magnesium sulfate, and evaporated under vacuum to obtain 1.8 g. of a solid residue, which was shown by nmr to consist in substantial part of the desired product, identical to the product of Example 7.

Example 10

N,N-Dimethyl-α-(3-pyridyl)-*m*-dioxane-5-methylamine

Eight g. of the enamine prepared above was dissolved in 60 ml. of methanol and chilled in an ice bath. The solution was stirred while 4.2 ml. of acetic acid in 15 ml. of methanol was added to it, and then

5.9 g. of sodium cyanoborohydride was added in one portion and the mixture was stirred for 30 minutes in an ice bath. A small portion of additional acetic acid was added to check for complete consumption of the hydride, and the mixture was then evaporated under vacuum to remove the solvent. The residue was dissolved in water, made basic with ammonium hydroxide and extracted four times with 75 ml. portions of dichloromethane. The combined organic layers were washed with saturated aqueous sodium chloride, dried over magnesium sulfate, filtered and evaporated under vacuum to obtain 7.7 g. of impure product, found by nmr analysis to consist substantially of the desired compound, identical to the product of Example 7.

## Example 11

N,N-Dimethyl-α-(3-pyridyl)-*m*-dioxane-5-methylamine, hydrochloride

The impure product prepared in Example 10 was dissolved in 40 ml. of acetone, and to it was added dropwise with stirring 2.88 ml. of concentrated hydrochloric acid dissolved in 3 ml. of acetone. The mixture was stirred for 2 hours, and was filtered to obtain 5.4 g. of crude hydrochloride. The crude product was dissolved in denatured ethanol, concentrated to 60 ml., stirred, seeded, and stored overnight at ambient temperature to obtain 3.8 g. of pure hydrochloride product, m.p. 196-201 *dec*. A second crop of 0.45 g. was obtained.

The following example illustrates the resolution of the *d*,*l*-mixture obtained from the above

examples to isolate the preferred l-isomer, in the form of its hydrochloride salt, the most preferred form of the products of this invention.

Example 12

l-N,N-Dimethyl-α-(3-pyridyl)-m-dioxane-5-methylamine, monohydrochloride

A 15 g. portion of N,N-dimethyl-α-(3-pyridyl)-m-dioxane-5-methylamine was mixed with 180 ml. of methyl ethyl ketone and 15.7 g. of l-camphorsulfonic acid. The mixture was heated to form a solution, and was filtered into a flask, where it was stirred vigorously at ambient temperature for 2 hours. The solids were removed by filtration and were vacuum dried to obtain 28.2 g. of the l-camphorsulfonic acid salt of the d,l mixture of the dioxane compound.

The solids, m.p. 154-158°, were dissolved in 375 ml. of methyl ethyl ketone and 18.8 ml. of methanol with heating, and the solution was filtered hot into a clean vessel, where it was stirred at ambient temperature for 7 hours and was seeded with authentic l-camphorsulfonic acid salt of l-N,N-dimethyl-α-(3-pyridyl)-m-dioxane-5-methylamine, when the temperature of the solution approached ambient. The mixture was filtered again, and the filtrate was stirred overnight at ambient temperature and filtered again to obtain a total of 10.0 g. of l-camphorsulfonic acid salt of l-N,N-dimethyl-α-(3-pyridyl)-m-dioxane-5-methylamine, m.p. 169-170°.

A 101 g. portion of the above l,l-salt, obtained from large-scale preparations similar to the

above, was dissolved in 320 ml. of dichloromethane and mixed with 32 ml. of aqueous concentrated ammonium hydroxide. It was necessary to add some additional base to aid in the separation of the layers. The organic layer was separated, the aqueous layer was washed twice with 50 ml. portions of dichloromethane, and the combined organic layers were washed twice with 50 ml. portions of dilute aqueous sodium chloride solution and then dried over magnesium sulfate. The organic solution was filtered and was evaporated under vacuum to obtain 36.5 g. of the desired l-N,N-dimethyl-α-(3-pyridyl)-m-dioxane-5-methylamine.

The above was dissolved in 170 ml. of acetone, and the solution was stirred and cooled in an ice bath while 13.7 ml. of concentrated hydrochloric acid in 15 ml. of acetone was added dropwise. The mixture was then stirred an additional 1.5 hours in the ice bath, and was filtered. The filter cake was washed with cold acetone, and the solids were air dried to obtain 39.6 g. of the desired product, identified by nmr in $D_2O$ as follows: δ8.63 (m, 2H); 8.03 (m, 1H); 7.60 (dd, 1H); 4.88 (m, 3H); 2.78 (br s, 1H); 2.78 (s, 6H).

CLAIMS

1. A process for preparing a compound of formula (I):

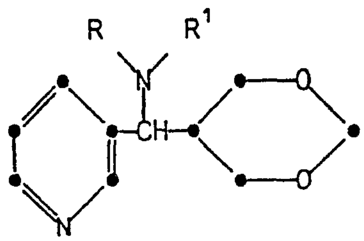

wherein R and $R^1$ are independently $C_1$-$C_4$ alkyl, or a pharmaceutically-acceptable, acid-addition salt thereof;

which process comprises reacting a pyridylcarbonyldioxane of the formula

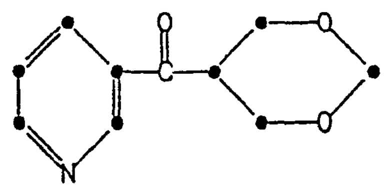

with a secondary amine of formula

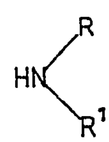

in the presence of titanium tetrachloride to prepare an enamine of the formula

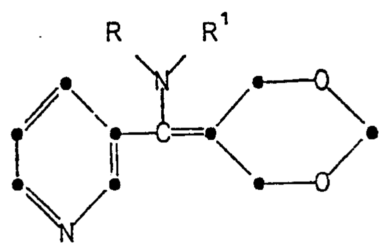

and reducing the enamine.

2. A process according to claim 1, wherein R and $R^1$ are methyl.

3. A compound of formula (I) whenever prepared by a process according to claim 1 or 2.

4. A pyridylcarbonyldioxane of the formula